# EUROPEAN PATENT APPLICATION

(11) **EP 4 644 536 A1**
(43) Date of publication of application: **05.11.2025**
(21) Application number: 23908932.9
(22) Date of filing: 28.04.2023
(51) Int. Cl.: C12N 5/0775, A61K 35/28, A61P 19/08, A61L 27/38, C12N 5/077, C12Q 1/02, G01N 33/68

(54) **STEM CELL, PREPARATION METHOD THEREFOR AND USE THEREOF, AND METHOD FOR TREATING BONE DEFECT**

(30) Priority: 30.12.2022 CN 202211737365
(71) Applicant: Northwestern Polytechnical University, Xi'an, Shanxi 710000 (CN)
(72) Inventor: QIU, Qiang, Shaanxi 710000 (CN); HUANG, Jinghui, Shaanxi 710000 (CN); QIN, Tao, Shaanxi 710000 (CN); WANG, Wen, Shaanxi 710000 (CN)
(74) Representative: Murgitroyd & Company
(86) International application number: PCT/CN2023/091457
(87) International publication number: WO 2024/138982

(57) **Abstract**

A stem cell, a preparation method therefor and a use thereof, and a method for treating a bone defect. A new stem cell is identified and separated, and the stem cell has strong osteogenesis and chondrogenic differentiation capabilities, is stronger than traditional bone mesenchymal stem cells in respect of osteogenic capability, and can thus be used for bone defect repair of animals or humans. The stem cell can be used for effectively and rapidly treating a bone defect, thereby shortening the bone repair time, and remarkably improving a prognosis effect.

## Description

The present disclosure claims priority of Chinese Patent Application No. 202211737365.3, filed on December 30, 2022, and entitled "Stem cell, preparation method therefor and use thereof, and labeled composition and use thereof", the disclosure of which is incorporated herein by reference in its entirety.

### TECHNICAL FIELD

The present disclosure relates to the technical field of stem cells, and in particular, to a stem cell, a preparation method therefor and use thereof, and a method for treating bone defects.

### BACKGROUND

Severe defects in bone structures caused by trauma, congenital malformations, or extensive tumor surgery usually require surgical reconstruction. Bone defects have been a major concern in trauma orthopedics, while bone defects can also lead to bone nonunion, making the patient's prognosis even less satisfactory. This will not only undermine the confidence of patients in overcoming the disease, but also make the repair of bone defects more and more concerned. At present, the first choice for the treatment of bone defect is autogenous bone or allogenic bone transplantation. Autogenous bone transplantation has low immunogenicity, but its materials are limited, bone harvesting is invasive, and complications may occur after bone harvesting, while allogenic bone transplantation is limited in its wide clinical application due to the frequent immune responses.

### SUMMARY

Thus, stem cell-based regenerative medicinal treatment is expected to become an important direction for the next treatment.

In view of this, the present disclosure provides a stem cell, a preparation method therefor and use thereof, and a method for treating bone defects. The present disclosure aims to provide a stem cell with strong bone repair ability.

Embodiments of the present disclosure are implemented as follows:

In a first aspect, the present disclosure provides a stem cell, and a marker expressed by the stem cell includes CX43 and FGFR2.

Optionally, in some embodiments of the present disclosure, the stem cells are CX43 positive and FGFR2 positive.

Optionally, in some embodiments of the present disclosure, the stem cells include a genetic marker selected from the group consisting of: FKBP11, PTN, TNN, TNC, OMD, ACTA2, MARCKS, and a combination thereof.

Optionally, in some embodiments of the present disclosure, the stem cells are mesenchymal stem cells.

Optionally, in some embodiments of the present disclosure, the stem cells are derived from human stem cells or animal stem cells.

Optionally, in some embodiments of the present disclosure, the animal stem cells include any one of deer-derived stem cells and murine-derived stem cells.

Optionally, in some embodiments of the present disclosure, the deer-derived stem cells are derived from one or two of a mesenchymal tissue of antler blastemas or a mesenchymal tissue at the top of antler growth center.

Optionally, in some embodiments of the present disclosure, the murine-derived stem cells are derived from mouse P3 digit tip regeneration tissue.

Optionally, in some embodiments of the present disclosure, the stem cells are induced by human stem cells.

In a second aspect, the present disclosure provides a method of preparing a stem cell. The method incluses steps as follows:
providing a cell population sample and an antibody composition, the antibody composition includes an anti-CX43 antibody and an anti-FGFR2 antibody; and
staining the cell population samples with the antibody composition, and isolating cells positive for CX43 and positive for FGFR2 from the cell population sample to obtain stem cells.

Optionally, in some embodiments of the present disclosure, the cell population sample is derived from animal stem cells or human stem cells.

Optionally, in some embodiments of the present disclosure, the animal stem cells are derived from one or more of the mesenchymal tissue of antler blastemas, the mesenchymal tissue at top of antler growth center and mouse P3 digit tip regeneration tissue.

Optionally, in some embodiments of the present disclosure, the step of "staining the cell population sample with the antibody composition, and isolating cells positive for both CX43 and FGFR2 from the cell population sample to obtain stem cells" includes:
staining the cell population sample with one of the anti-CX43 antibody and the anti-FGFR2 antibody, and detecting and isolating positive cells in the cell population sample by flow cytometry to obtain primary screening cells; and
staining the primary screening cells with another one of the anti-CX43 antibody and the anti-FGFR2 antibody, and detecting and isolating positive cells in the primary screening cells by flow cytometry to obtain stem cells.

In a third aspect, the present disclosure further proposes use of stem cells in preparing a product for treating bone defects, wherein the stem cells include the stem cells as described above, or the stem cells include stem cells prepared by the method as described above, or the stem cells include the stem cells expressing the labeled composition as described above;
wherein the product for treating bone defects include drugs for treating bone defects, materials for treating bone defects, or kits for treating bone defects.

Optionally, in some embodiments of the present disclosure, the drugs for treating bone defects include the stem cells and a pharmaceutically acceptable carrier.

Optionally, in some embodiments of the present disclosure, the materials for treating bone defects include any one of tissue engineering bone materials, bone defect scaffold materials, and bone defect composite repair materials.

Optionally, in some embodiments of the present disclosure, the materials for treating bone defects include the stem cells and implantation materials, and the implantation materials include one or more of natural polymers, synthetic polymers, biomedical metal materials, inorganic materials.

Optionally, in some embodiments of the present disclosure, the bone defect scaffold materials include the stem cells and medical bone materials, and 1×10⁴ to 1×10⁵ of the stem cells are inoculated per cubic millimeter of the medical bone materials.

Optionally, in some embodiments of the present disclosure, application subject of the product for treating bone defects is a human or animal.

In a fourth aspect, the present disclosure further provides a method of treating bone defects, wherein the method includes administering to to a subject drugs, materials, or kits including an effective amount of stem cells, wherein the stem cells include the stem cell as described above, or the stem cells include stem cells prepared by the method as described above.

### BENEFICIAL EFFECT

In the technical solutions provided in the present disclosure, a new type of stem cells is identified and isolated, and the stem cells have stronger osteogenic and chondrogenic differentiation abilities, and its osteogenic ability is stronger than that of conventional bone marrow mesenchymal stem cells (BMSCs), and can be used for repairing bone defects of animals or humans. By using the stem cells, bone defects can be effectively and quickly treated, the bone repair time can be shortened, and the prognosis effect can be significantly improved.

### BRIEF DESCRIPTION OF THE DRAWINGS

In order to more clearly describe the technical solutions in the specific embodiments of the present disclosure, hereinafter, the appended drawings used for describing the specific embodiments will be briefly introduced. Apparently, the appended drawings described below are only directed to some embodiments of the present disclosure, and for a person skilled in the art, without expenditure of creative labor, other drawings can be derived on the basis of these appended drawings.
FIG. 1 is a graph showing the morphological effect of stem cells according to embodiments of the present disclosure;
FIG. 2 is a violin plot of genetic marker expression of stem cells according to embodiments of the present disclosure;
FIG. 3 is a 10× single-cell transcriptomic profile of regeneration tissue of antler blastemas and mouse P3 digit tip regeneration tissue;
FIG. 4 is a graph showing qualitative test result of osteogenic and chondrogenic differentiation induction abilities in Experimental Example 1;
FIG. 5 is a graph showing quantitative PCR detection results of osteogenic and chondrogenic differentiation induction efficiency in Experimental Example 1;
FIG. 6 is a graph showing the monoclonal ability test results of the stem cells obtained in the Examples of Experimental Example 1 and Comparative Examples 2 to 4;
FIG. 7 is a graph showing the results of newly formed bone under renal capsule stained with ponceau red in Experimental Example 2;
FIG. 8 is a graph comparing the statistical results of newly formed bone area in Experimental Example 2;
FIG. 9 is a Micro-CT map of the lateral condyle bone of the defect group, comparative example treatment group and stem cell treatment group in Experimental Example 3;
FIG. 10 is a statistical graph of newly formed bone volume fraction at the defect site in the defect group, comparative example treatment group and stem cell treatment group in Experimental Example 3;
FIG. 11 is a statistical graph of the number of newly formed bone trabeculae at the defect site in the defect group, comparative example treatment group and stem cell treatment group in Experimental Example 3;
FIG. 12 is a HE staining diagram of the lateral condyle bone of the defect group, comparative example treatment group and stem cell treatment group in Experimental Example 3; and
FIG. 13 is a Saf-O/Fast Green staining diagram of the defect group, comparative example treatment group and stem cell treatment group in Experimental Example 3.

### DETAILED DESCRIPTION

Hereinafter, technical solutions in examples of the present disclosure will be clearly and completely described with reference to the appended drawings in examples of the present disclosure. Apparently, the described examples are part of, but not all of, the examples of the present disclosure. All the other examples, obtained by a person skilled in the art on the basis of the examples in the present disclosure without expenditure of creative labor, belong to the protection scope of the present disclosure. In addition, it should be understood that specific embodiments described herein are only used to illustrate and explain the present disclosure, and are not intended to limit the present disclosure.

Unless otherwise defined, all professional and/or scientific terms used herein have the same meaning as those familiar to a person skilled in the art. In addition, any methods and materials similar or equivalent to those described herein can be applied to the present disclosure. The preferred embodiments and materials described herein are exemplary only, but are not intended to limit the content of the present disclosure.

In addition, in the description of the specification, the term "comprising" means "including but not limited to". Various embodiments of the present disclosure may be presented in a range format. It should be understood that the description in range format is merely for convenience and brevity and should not be construed as an inflexible limitation on the scope of the present disclosure. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range. Whenever a numerical range is indicated herein, it is meant to include any cited numeral (fractional or integral) within the indicated range.

Unless otherwise specified, the raw materials and reagents used in the following examples are all commercially available products, or can be prepared by methods known in the art.

In the present disclosure, the term "and/or" describes an association relationship for describing associated objects and represents that three relationships may exist. For example, A and/or B may represent the following three cases: only A exists, both A and B exist, and only B exists. Wherein A, B may be singular or complex.

In the present disclosure, "at least one" means one or more, and "a plurality of" means two or more. "At least one", "at least one of the following items (pieces)", or a similar expression thereof refers to any combination of these items, including any combination of singular items (pieces) or plural items (pieces). For example, "at least one item (piece) of a, b, or c" or "at least one item (piece) of a, b, and c" may represent a or b or c; a-b (i.e., a and b); a-c, b-c, or a-b-c, where a, b, and c each may be singular or plural.

In the present disclosure, the bone defect includes bone defects caused by trauma, surgery, or disease. Bone defects caused by diseases include bone-related diseases caused by diabetes, rheumatoid arthritis, autoimmune encephalomyelitis, systemic lupus erythematosus, multiple sclerosis, periodontitis, inflammatory bowel disease, mucositis, colitis, or sepsis.

The technical solutions of the present disclosure are as follows:

According to a first aspect, embodiments of the present disclosure provide stem cells, which are pluripotent stem cells. They have the following characteristics: the stem cells have a strong osteogenic and chondrogenic differentiation ability, and the osteogenic ability is stronger than that of conventional bone marrow mesenchymal stem cells (BMSCs), and can be used for repairing bone defects of animals or humans. By using the stem cells, bone defects can be effectively and quickly treated, the bone repair time can be shortened, and the prognosis effect can be significantly improved.

The marker expressed by the stem cells disclosed herein includes CX43 and FGFR2. The marker expression may refer to stem cells can express specific marker genes, or express specific marker proteins, or express marker genes and proteins. The stem cells can be identified and seprated by combination of the above markers, and the stem cells seprated based on the combination of the above markers have stronger osteogenic and chondrogenic differentiation ability, and their osteogenic abilities are stronger than those of traditional BMSCs. It can be understood that the determination of whether the stem cells express each marker can be performed, for example, by existing known methods such as gene chip arrays and polymerase chain reactions (reverse transcriptase PCR, real-time PCR, existing PCR), in terms of gene expression. In addition, with regard to protein expression, for example, it can be carried out by existing known methods such as FACS analysis (flow cytometry), enzyme-linked immunosorbent assay (ELISA) using antibodies specifically binding to each marker protein. In any case, cells that do not express each marker gene or protein are used as negative controls to determine the presence or absence of expression and the level of expression. Specifically, in cell samples, cells that are positive for both CX43 and FGFR2 are stem cells as set forth herein.

### Specifically:

CX43 refers to recombinant connexin 43, which is located on the outer surface of the cell membrane of the stem cells in the present disclosure, and this protein plays an important role in embryonic development. Mutations in this gene can lead to developmental deformaties in bone.

FGFR2 refers to fibroblast growth factor receptor 2. FGFR2 protein is located on the outer surface of the cell membrane of stem cells of the present disclosure. It is encoded by the FGFR2 genes located on chromosome 10, which plays an important role in tissue repair, bone and angiogenesis.

During separation, cells that are positive for both CX43 and FGFR2 are selected to obtain the stem cells described in the present disclosure. Moreover, CX43 and FGFR2 are located on the outer surface of the cell membrane, and the antibody staining is more accurate, more effective and faster.

Furthermore, the stem cells further include at least one of the following genetic markers: FKBP11 (FKBP prolyl isomerase 11), PTN (pleiotrophin), TNN (tenascin N), TNC (tenascin C), OMD (osteomodulin), ACTA2 (actin alpha 2, smooth muscle), and MARCKS (myristoylated alanine-rich C-kinase substrate). Further identification of stem cells isolated based on the above markers to determine whether they have at least one of the above genetic markers is helpful for further identification of stem cells. Meanwhile, based on the above characteristics, the stem cells are capable of secreting expression products of at least one of FKBP11, PTN, TNN, TNC, OMD, ACTA2, and MARCKS, so they have the functions of these expression products. It should be noted that the above-mentioned genetic markers include corresponding genetic markers of humans or animals or the genetic markers whose nucleotide sequence homology is not less than 98%. Taking MARCKS as an example, its nucleotide sequence includes, but is not limited to, the nucleotide sequence of a human MARCKS gene, a nucleotide sequence with homology of not less than 98% with the nucleotide sequence of the human MARCKS gene, a nucleotide sequence of the animal MARCKS gene, and a nucleotide sequence with homology of not less than 98% with the nucleotide sequence of the animal MARCKS gene. Among them, animals include, but are not limited to, deer, mice, and the like. Depending on the source of the stem cells, genetic markers contained therein correspond to the corresponding genetic markers of an organism source or genetic markers with nucleotide sequence homology of not less than 98% with the corresponding genetic markers of the organism source. Under the condition of knowing which organism the stem cells originate from, the person skilled in the art can obtain their sequence information (and sequences with homology of not less than 98%) and characteristics through conventional methods.

In a specific embodiment:
the nucleotide sequence of MARCKS includes a sequence having accession number XM_043889887.1 on NCBI and a sequence with homology of not less than 98% to the sequence (for example, a nucleotide sequence as shown in SEQ ID NO.1);
the nucleotide sequence of FKBP11 includes a sequence having accession number XM_006054550.3 on NCBI and a sequence with homology of not less than 98% to the sequence (for example, a nucleotide sequence as shown in SEQ ID NO.2);
the nucleotide sequence of PTN includes a sequence having accession number XM_043873485.1 on NCBI and a sequence with homology of not less than 98% to the sequence (for example, a nucleotide sequence as shown in SEQ ID NO.3);
the nucleotide sequence of TNN includes a sequence having accession number XM_043924613.1 on NCBI and a sequence with homology of not less than 98% to the sequence (for example, a nucleotide sequence as shown in SEQ ID NO.4);
the nucleotide sequence of OMD includes a sequence having accession number XM_043453757.1 on NCBI and a sequence with homology of not less than 98% to the sequence (for example, a nucleotide sequence as shown in SEQ ID NO.5);
the nucleotide sequence of ACTA2 includes a sequence having accession number XM_043476084.1 on NCBI and a sequence with homology of not less than 98% to the sequence (for example, a nucleotide sequence as shown in SEQ ID NO. 6); and
the nucleotide sequence of TNC includes a sequence having accession number XM_005251975.5 on NCBI and a sequence with homology of not less than 98% to the sequence (for example, a nucleotide sequence as shown in SEQ ID NO.7).

The protein expressed by MARCKS is a myristoylated alanine-rich C-kinase substrate, which is believed to be related to cell movement, phagocytosis, membrane transport and mitosis, and plays an important role in limb regeneration. MARCKS is located on the inner membrane of the cell and is highly expressed in the stem cells. In practical applications, MARCKS can also be used as a marker to identify the stem cells. The protein expressed by FKBP11 is FKBP prolyl isomerase 11, which is related to bone development. The protein expressed by PTN is heparin-binding growth factor, and the protein encoded by this gene plays an important role in cell growth and survival, cell migration, angiogenesis and tumorigenesis. The protein expressed by TNN refers to tendon glycoprotein-W, which is involved in multiple biological processes, including the generation of neurons, the negative regulation of classical Wnt signaling pathways of osteoblast differentiation, and the negative regulation of osteoblast differentiation, and is related to the formation of cartilage. The protein expressed by TNC is tenascin-C, which constitutes the extracellular matrix of the tissue and participates in multiple biological processes such as bone and nerve development. The protein expressed by OMD is osteomodulatory protein, which is mainly involved in the formation of intercellular matrix and is related to bone mineralization. The protein expressed by ACTA2 is α-smooth muscle actin, which is a highly conserved protein that participates in cell movement, structural composition and intercellular signaling.

In some embodiments of the present disclosure, the stem cells are mesenchymal stem cells. In the present disclosure, the mesenchymal stem cells refer to cells having the ability to differentiate into mesenchymal cells such as osteocytes, cardiomyocytes, chondrocytes, tendon cells, and adipocytes and can proliferate while maintaining the differentiation ability, for example, mesenchymal stem cells derived from bone marrow, adipose tissue, blood, periosteum, dermis, umbilical cord, placenta, amnion, chorion, deciduous membrane, muscle, endometrium, dermis, dental follicle, periodontal membrane, dental pulp or tooth germ, preferably include mesenchymal stem cells derived from umbilical cord, adipose tissue or bone marrow. It can be understood that "derived from" means that the above mentioned cells have been acquired, grown or manipulated in vitro from tissue serving as a supply source thereof.

In some embodiments of the present disclosure, the stem cells are derived from human stem cells or animal stem cells. Specifically, the stem cells proposed in the present disclosure may be isolated directly from human stem cells or animal stem cells, or may be obtained via artificial induction. As an example, the method of obtaining the stem cells may include, but is not limited to, in some embodiments, obtaining the stem cells from the mesenchymal tissue of antler blastemas or the mesenchymal tissue at the top of growth center. In a specific procedure, only limited invasive surgery is needed to obtain tissues, and then the stem cells can be isolated. The operation is simple, and the mesenchymal stem cells obtained from the tissues can be cultured and passaged without differentiation. Therefore, the stem cells can be efficiently recovered under GMP conditions after simple optimization, and the source is stable, which significantly reduces the medical burden. In other embodiments, the stem cells are obtained from the mouse P3 digit tip regeneration tissue, and the source is stable and easy to obtain. In still other embodiments, the stem cells may also be induced from human stem cells by chemical induction or gene editing. For example, human stem cells are transformed by CRISPER-Cas9 technology to have similar chondrogenic and osteogenic abilities as the stem cells in the present disclosure. Compared with animal-derived stem cells, human stem cells are more suitable for the treatment of human bone defects and have higher safety.

It should be noted that, in this text, the specific experimental methods and experimental raw materials involved in the technical transformation of CRISPER-Cas9, tissue acquisition, cell staining, etc., are all carried out with reference to the conventional methods in the art, and will not be described in detail here.

In a second aspect, the present disclosure further provides a method of preparing stem cells. The method includes steps as follows:
step S10, providing a cell population sample and an antibody composition, wherein the antibody composition includes an anti-CX43 antibody and an anti-FGFR2 antibody;
step S20, staining the cell population sample with the antibody composition; and
step S30, isolating cells positive for both CX43 and FGFR2 from the cell population sample to obtain stem cells.

In a third aspect, the present disclosure further provides use of stem cells as described above in preparing a product for treating bone defects. The characteristics and isolation methods of the stem cells have been previously described and will not be repeated here.

The product for treating bone defects include drugs for treating bone defects, materials for treating bone defects, or kits for treating bone defects.

The drugs for treating bone defects refer to drugs for treating and repairing bone defects, which have the function of treating and repairing bone defects. Specifically, the drugs include drugs that promote bone regeneration, and drugs for treating bone defects include the stem cells. For example, a composite bio-gel for repairing bone defects, or a biological preparation for promoting osteogenic differentiation of bone marrow mesenchymal stem cells and the like. Specifically, the composite bio-gel is obtained by planting the stem cells on a bioactive gel after in vitro amplification and culture, the biological preparation contains the stem cells. The preparation method of the drugs can refer to the preparation of common drugs for treating bone defects in the art, which will not be described in detail here.

The drugs for treating bone defects may also include a pharmaceutically acceptable carrier. The term "pharmaceutically acceptable carrier" refers to a diluent, auxiliary material, excipient, or vehicle. The term "pharmaceutically acceptable carrier" is, within the scope of sound medical judgment, suitable for contact with the tissues of human beings and animals without excessive toxicity, irritation, allergic response, or other problem or complication, commensurate with a reasonable benefit/risk ratio.

The materials for treating bone defects refer to materials for treating, repairing bone defects. For example, the materials may be tissue engineering bone materials for bone defect regeneration and repairing, bone defect scaffold materials, bone defect composite repair materials, functional composite scaffold materials, and the like. The materials include stem cells and implantation materials, and the implantation materials can be selected from but not limited to at least one of natural polymers, synthetic polymers, biomedical metal materials, and inorganic materials. As as example, the bone defect scaffold materials may include the stem cells and medical bone materials. Specifically, the bone defect scaffold materials may be obtained by uniformly inoculating 1×10⁴ to 1×10⁵ of the stem cells per cubic millimeter of the medical bone materials.

The kit refers to a kit that is capable of preparing drugs or materials for treating bone defects. In some embodiments of the present disclosure, the kit includes the stem cells and a carrier. The carrier may be a medical sponge, a medical dressing, a gel composition that can be made into a gel, or the like. For example, in a specific embodiment, the kit includes 2 to 10 mL of stem cell suspension (cell concentration was 10⁴ to 10⁶/µL) and gelatin sponge. The method of using the kit is as follows: adding the stem cell suspension into the gelatin sponge prior to use to form scaffold filling materials, filling the scaffold filling materials at the bone defects, and stitching the muscle and skin at the bone defects.

The stem cells proposed in the present disclosure can be applied to humans or animals, and accordingly, the application subject of product for treating bone defects can be both humans and animals, which has broad development prospects.

In a fifth aspect, the present disclosure further provides a labeled composition, which includes but is not limited to CX43 and FGFR2. The labeled composition is capable of screening and identifying stem cells. The stem cells screened or identified based on the labeled composition have stronger osteogenic and chondrogenic differentiation ability, and its osteogenic ability is stronger than that of conventional bone marrow mesenchymal stem cells (BMSCs). By using the stem cells, bone defects can be effectively and quickly treated, the bone repair time can be shortened, and the prognosis effect can be significantly improved.

It can be understood that the CX43 and FGFR2 may be corresponding genetic features or expression proteins. For example, in some embodiments, the nucleotide sequence of the FGFR2 includes a sequence having accession number XM_043926212.1 on NCBI and a sequence with homology of not less than 98% to the sequence (for example, a nucleotide sequence as shown in SEQ ID NO. 8); the nucleotide sequence of the CX43 includes a sequence having accession number XM_OU343105.1 on NCBI and a sequence with homology of not less than 98% to the sequence (for example, a nucleotide sequence as shown in SEQ ID NO. 9).

In addition, the labeled composition further includes an expression product of at least one of FKBP11, PTN, TNN, TNC, OMD, ACTA2, and MARCKS. The stem cells isolated based on the above markers are further identified to determine whether they have at least one of the above genetic markers, which is helpful for further identification of stem cells.

In a sixth aspect, the present disclosure further proposes use of the labeled composition as described above in preparing a kit for isolating or identifying stem cells.

Specifically, the kit includes anti-Connexin 43 (10 µL), anti-FGFR2 (10 µL), fluorescently labeled secondary antibody FITC (10 µL), and fluorescently labeled secondary antibody Cy3 (10 µL).

The method of using this kit is as follows: isolating antler mesenchymal stem cells from 5-day-old antler tissues, and incubating the above antler mesenchymal stem cells with primary antibodies anti-Connexin 43 and anti-FGFR2 (1:200) for 1 hour; washing the cells with PBS for three times, diluting the two fluorescently labeled secondary antibodies at a ratio of 1:200, and mixing and incubating for 1 hour. Then detecting the proportion of double-fluorescence positive cells (cells emitting fluorescent signals) by flow cytometry, and isolating the double-positive cells by flow cytometry to obtain primary screening cells.

The technical solutions and the technical effects of the present disclosure are described in detail below by means of specific examples. The following examples are only some examples of the present disclosure and do not specifically limit the present disclosure.

### Examples Preparation of stem cells

(1) Preparation of antler mesenchymal stem cells: Antler samples on the fifth day of the regeneration process, and dust and blood stains on the surface of the antlers were removed by using sterile gauze. After skin was prepared, and disinfection treatment was carried by using 75% alcohol. Then regeneration tissue with a thickness of 3 cm of the antler blastemas was cut by using a disposable sterile tissue slice blade, which was minced with a blade and digested with collagenase I (100 µg/ml)/collagenase II (100 µg/ml)/collagenase IV (30 µg/ml) solution (Gibco, 17100017, 17101015 and 17104019) at 37 °C for 60 minutes (or until the tissue was completely digested), followed by shaking intermittently to obtain a single cell suspension. The single cell suspension was filtered through a 70 µm cell filter and a 40 µm cell filter (Corning Falcon, 352350 and 352340) in turn and centrifuged at 500xg for 5 minutes to obtain cell particles. The cell particles resuspended with PBS and treated with RBC lysis buffer (Solarbio, R1010) to remove red blood cells. Followed by washing twice with PBS, resuspending with 0.04% ultrapure BSA (Invitrogen) to obtain cells, and the cells were counted by using a Countess automated cell counter (Bio-Rad).

The cells were washed with flow sorting buffer at 0 to 4°C, and centrifuged at 1000g for 5 minutes. The steps were repeated twice to obtain antler mesenchymal stem cells.

### (2) Isolation of stem cells:

a. The antler mesenchymal stem cells were subjected to immunofluorescence staining by using a primary antibody anti-Connexin 43 (biorbyt, orb995524); then the proportion of positive cells (cells emitting fluorescent signals) was detected by flow cytometry, and CX43-positive cells were isolated by flow cytometry to obtain primary screening cells.
b. The antler mesenchymal stem cells were then subjected to immunofluorescence staining by using an anti-FGFR2 (LSBio, LS-C214232-200) solution; the proportion of positive cells (cells emitting fluorescent signals) was detected by flow cytometry, and FGFR2-positive cells were isolated by flow cytometry to obtain CX43⁺ FGFR2⁺ stem cells.

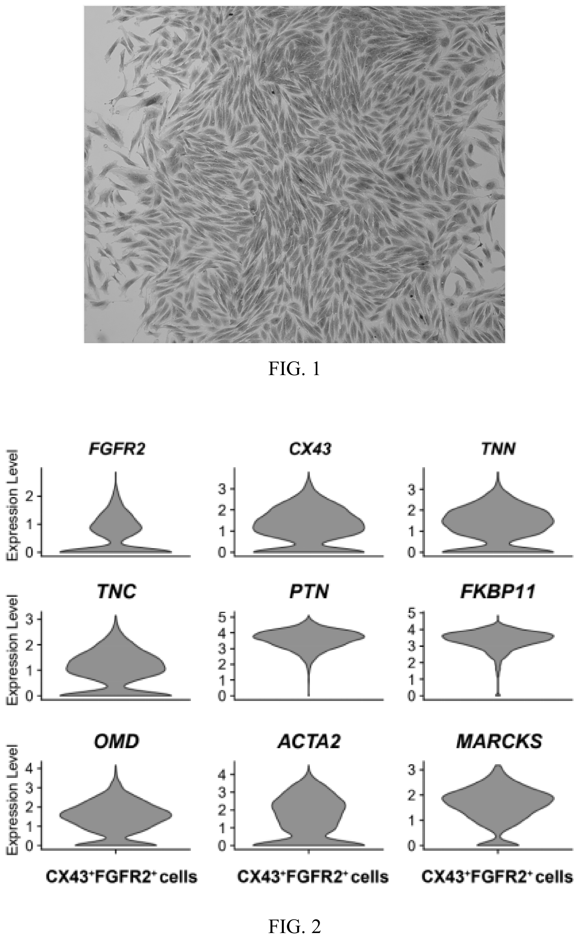

(1) The morphological effect of the CX43⁺FGFR2⁺ stem cells is shown in FIG. 1, from which it can beseen that the cells exhibit a typical long spindle shape.
(2) Detection of genetic marker

The CX43⁺FGFR2⁺ stem cells obtained in the examples were subjected to single-cell transcriptome sequencing by using the 10×genomic technique, and the sequencing data was 100 g. The sequencing data were aligned to the sika deer reference genome by using Cellranger software to quantify the expression of key genes.

After detection, the stem cells contain the following genetic markers: MARCKS having a nucleotide sequence as shown in SEQ ID NO.1, FKBP11 having a nucleotide sequence as shown in SEQ ID NO.2, PTN having a nucleotide sequence as shown in SEQ ID NO.3, TNN having a nucleotide sequence as shown in SEQ ID NO.4, OMD having a nucleotide sequence as shown in SEQ ID NO.5, ACTA2 having a nucleotide sequence as shown in SEQ ID NO.6, TNC having a nucleotide sequence as shown in SEQ ID NO.7.

In addition, the expression of the above-mentioned genetic markers is shown in FIG. 2, from which itcan be seen that the above-mentioned genetic markers are highly expressed in CX43⁺FGFR2⁺ stem cells.

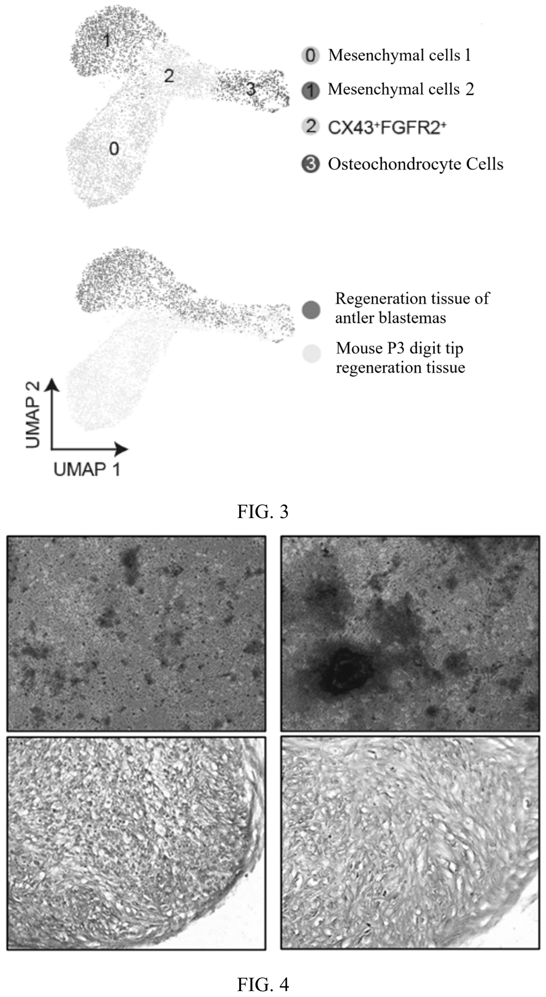

(3) Mouse P3 digit tip regeneration tissue was collected, and 10× single-cell transcriptome maps of the mouse P3 digit tip regeneration tissue and the regeneration tissue of the antler blastemas in step (1) of Example 1 were collected. The maps of the two were integrated by CCA method in the Seurat software, then the data was clustered by dimensionality reduction, and displayed by using the UMAP method. The results were shown in FIG. 3, it can be seen from the figure that the cell population of CX43⁺FGFR2⁺ stem cells is present in both regeneration tissue of antler blastemas and mouse P3 digit tip regeneration tissue.

### Comparative Example 1

Conventional BMSCs were used as Comparative Example 1.

### Comparative Example 2

The scheme of this comparative example is basically the same as Example 1, and the only difference is that in this comparative example:

in step (2), cells that were positive for CX43 and cells that were negative for FGFR2 were isolated by flow cytometry, and the isolated stem cells (CX43⁺FGFR2⁻ stem cells) were used as products for subsequent experiments.

### Comparative Example 3

This scheme of this comparative example is basically the same as Example 1, and the only difference is that in this comparative example:
in step (2), cells that were negative for CX43 negative and cells that were positive for FGFR2 were isolated by flow cytometry, and the isolated stem cells (CX43⁻FGFR2⁺ stem cells) were used as products for subsequent experiments.

### Comparative Example 4

The scheme of this comparative example is basically the same as Example 1, and the only difference is that in this comparative example:
in step (2), cells that were both negative for CX43 and FGFR2 were screened out, and the isolated stem cells (CX43⁻FGFR2⁻ stem cells) were used as products for subsequent experiments.

### Experimental Example 1

(1) The osteogenic and chondrogenic differentiation induction abilities of the isolated stem cells in the Examples and the isolated stem cells in Comparative Example 1 were detected. The detection method is as follows:

Detection of osteogenic differentiation ability: the stem cells were seeded in a 6-well plate, and differentiated for 28 days by using osteogenic differentiation induction medium (Cyagen Biosciences, GUXMX-90021) (changing the medium every 2 days). RNA was extracted, and RUNX2 and SP7 genes were detected quantitatively by qPCR. Meanwhile, the cells were stained by using alizarin red.

Detection of chondrogenic differentiation ability: 2.5×10⁵ stem cells were added to a 15 ml centrifuge tube, and centrifuging at 1000 rpm, then the cells were added to a cartilage differentiation induction medium (Cyagen Biosciences, GUXMX-90041) for culture. After 24 hours, chondrocytes were formed, and differentiation was continued for 3 weeks (changing the medium every 2 days). RNA was extracted, and SOX9 and COL2A1 genes were detected quantitatively by qPCR. Meanwhile, the chondrospheres were embedded, frozen, and cut into slices, and then the slices were stained with alcian blue.

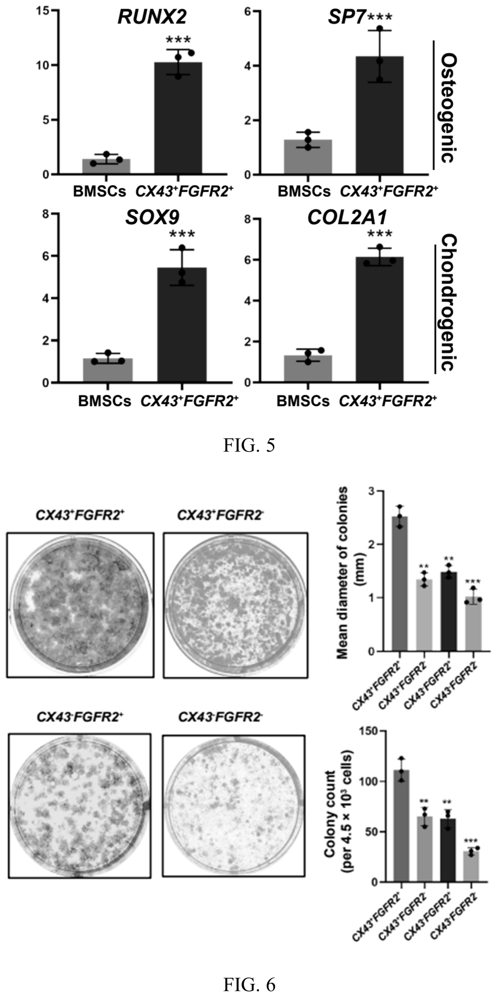

The results were shown in FIGs. 4 and 5. In FIG. 4, the alizarin red stained image in the detection of osteogenic differentiation ability and the alcian blue stained image in the detection of osteogenic differentiation ability were sequentially shown from top to bottom. Stem cells of Comparative Example 1 and Examples were shown from left to right.

As can be seen from FIG. 4, compared with BMSCs, CX43⁺FGFR2⁺ stem cells have stronger osteogenic and chondrogenic differentiation abilities. As can be seen from FIG. 5, the differentiation efficiency was quantified by quantitative real-time PCR (qPCR) analysis of osteogenic (RUNX2 and SP7) and chondrogenic (SOX9 and COL2A1) marker genes, and the quantification results were consistent with the results shown in FIG. 4, that is, compared with BMSCs, CX43⁺FGFR2⁺ stem cells had stronger osteogenic and chondrogenic differentiation abilities. This indicates that the stem cells isolated from the Examples showed osteogenic and chondrogenic differentiation induction abilities superior to the conventional BMSCs stem cells in Comparative Example 1.

(2) The monoclonal ability of the isolated stem cells in the Examples and the isolated stem cells in Comparative Examples 2 to 4 were detected. The detection method is as follows:

The stem cells were seeded in a 6-well plate (4.5×10³ cells/well), and 2 ml of DMEM medium (supplemented with 10% FBS and 1% penicillin/streptomycin solution) was added to each well, and incubated at 37° C in a 5% CO₂ cell incubator. The medium was changed every 2 days. On the 10th day, the cells were fixed and stained with crystal violet stain. The adherent colonies of more than 50 cells were quantified.

The results were shown in FIG. 6, compared with CX43⁻FGFR2⁻ stem cells, CX43⁺FGFR2⁻ stem cells, and CX43⁻FGFR2⁺ stem cells, the CX43⁺FGFR2⁺ stem cells showed higher colony forming efficiency, and there were more colonies with larger size, indicating that the CX43⁺FGFR2⁺ stem cells had stronger clonal expansion ability and had better effects when used for repairing bone defects.

### Experimental Example 2

The stem cells isolated in the Examples and the stem cells in Comparative Example 1 were subjected to subrenal transplantation and osteogenesis experiments. The experimental steps are as follows:

Providing 0.05 mL of matrigel (BD Matrigel, 356234), adding 5×10⁶ stem cells, and resuspending to obtain composite materials.

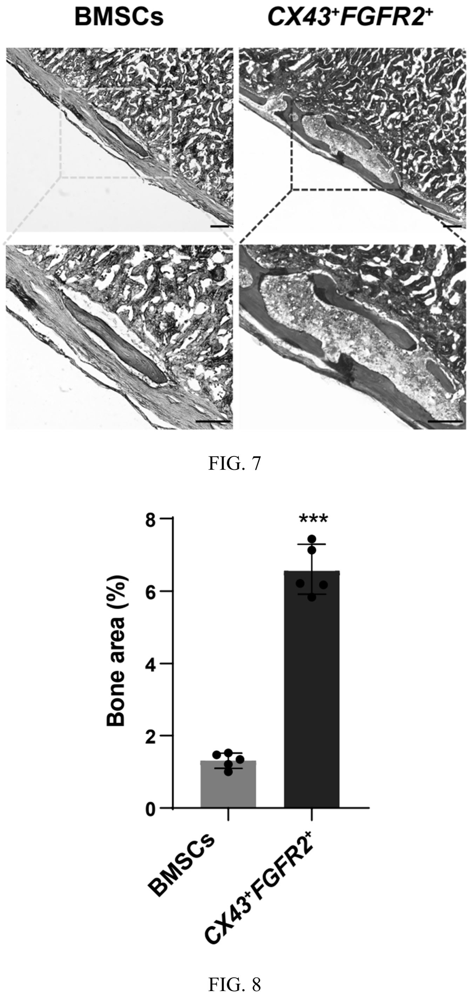

Ten immunodeficient mice (male, 8 to 10 weeks) were used and divided into 2 groups, i.e., BMSCs group and CX43⁺FGFR2⁺ group. The immunodeficient mice were anesthetized and disinfected, and a 1 cm incision was made on the right back with a scalpel to remove the right kidney. A small incision was made in the lower part of the kidney, and 5 µL of composite materials (containing 5×10⁵ cells) was implanted under the renal capsule with a pipette. The renal capsule incision was closed with a cauterizer, and the skin incision was closed. After 8 weeks, the animals were euthanized, and their kidneys were removed, fixed, dehydrated, frozen, cut into slices, and stained with ponceau red. The results were shown in FIG. 7 and FIG. 8, in which FIG. 7 is a graph showing the results of newly formed bone under renal capsule stained with ponceau red; and FIG. 8 is a graph comparing the statistical results of newly formed bone area.

As can be seen from FIG. 7 and FIG. 8, not only the stem cells isolated in the Examples were able to successfully form newly formed bone under renal capsule of the mouse, but also, compared with Comparative Example 1, the area of the newly formed bone formed in the Experimental Example corresponding to the stem cells isolated in the Examples is larger, indicating that the stem cells isolated in the Examples had stronger osteogenic ability compared with the conventional BMSCs.

### Experimental Example 3

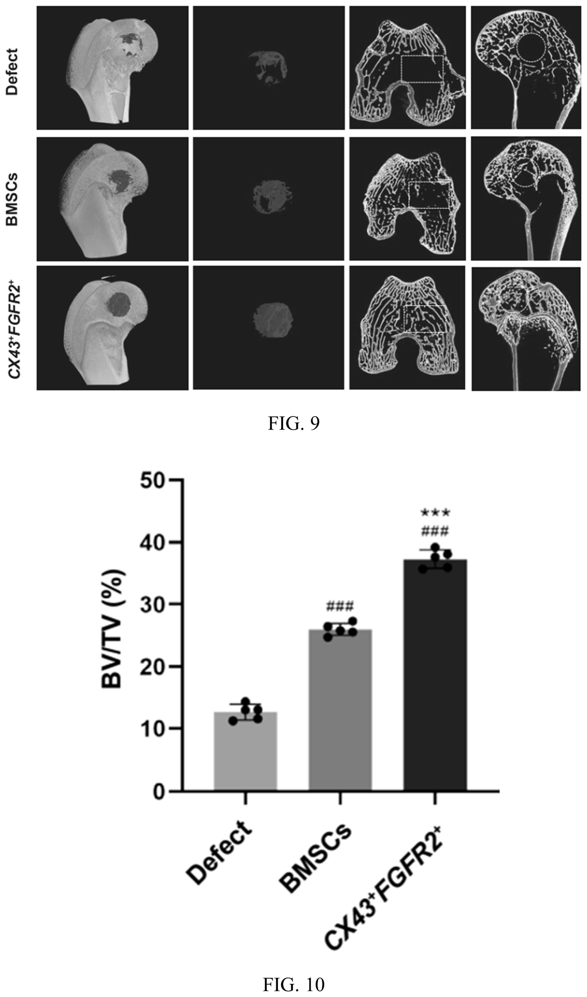

(1) A kit for treating bone defects was provided. The kit includes 2 mL of a stem cell suspension (cell concentration was 2×10⁴/µL) containing stem cells prepared in the Examples, and gelatin sponge (size: 2×2×0.2 cm, 4 pieces). Prior to use, 500 µL stem cell suspension was added to a piece of gelatin sponge to make a scaffold containing 1×10⁷ stem cells.
(2) A lateral femoral condyle bone defect model was established by using New Zealand male rabbits (15 rabbits, 2 months old). The specific method was to create a defect with a diameter of 8 mm and a depth of 6 mm in the right femoral lateral condyle of the rabbit by using an electric drill. Fifteen New Zealand male rabbits were randomly divided into three groups (5 rabbits in each group): defect group (defect), comparative treatment group (BMSCs) and stem cell treatment group (CX43⁺FGFR2⁺ stem cells). Among them: the defect group was only modeled without any treatment; the comparative treatment group was treated by filling a gelatin sponge containing 1×10⁷ BMSCs cells at the defect site; and the stem cell treatment group was treated by filling the scaffold prepared in step (1) at the defect site. Samples were taken after 8 weeks and then subjected to micro-CT detection. The results were shown in FIGs. 9-13, wherein:

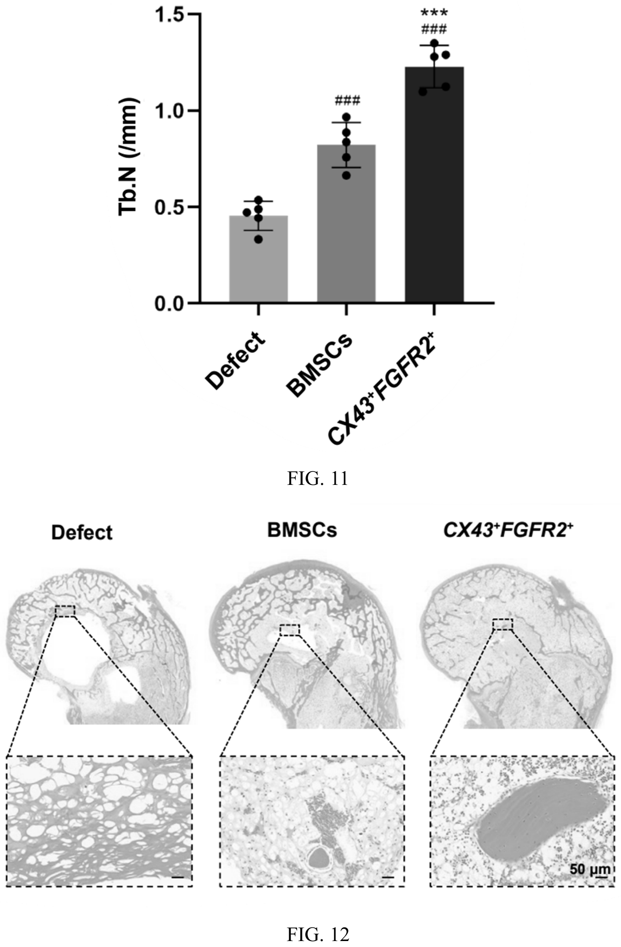

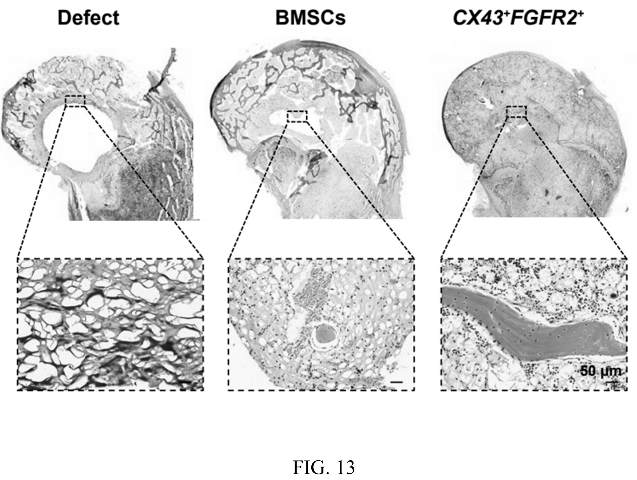

FIG. 9 is a Micro-CT map of the lateral condyle bone of the defect group, comparative example treatment group and stem cell treatment group in Experimental Example 3, FIG. 10 is a statistical graph of newly formed bone volume fraction at the defect site in the defect group, comparative example treatment group and stem cell treatment group in Experimental Example 3, FIG. 11 is a statistical graph of the number of newly formed bone trabeculae at the defect site in the defect group, comparative example treatment group and stem cell treatment group in Experimental Example 3; FIG. 12 is a HE staining diagram of the lateral condyle bone of the defect group, comparative example treatment group and stem cell treatment group in Experimental Example 3, and FIG. 13 is a Saf-O/Fast Green staining diagram of the defect group, comparative example treatment group and stem cell treatment group in Experimental Example 3.

Analysis of the results: in the CX43⁺FGFR2⁺stem cell treatment group, newly formed bone was successfully formed at the defect site of the right femoral lateral condyle of the rabbit. Further, it can be seen from FIGs. 9 to 10 that, in a same period of time, the newly formed bone volume fraction at the defect site in the CX43⁺FGFR2⁺stem cell treatment group was significantly higher than that in the BMSCs treatment group, and the number of newly formed bone trabeculae at the defect site in the CX43⁺FGFR2⁺stem cell treatment group was significantly higher than that in the BMSCs treatment group. It can be seen from FIGs. 12 and 13 that the newly formed bone is well combined with adjacent natural tissue and has a normal morphology. This indicates that the stem cells provided in the present disclosure have stronger osteogenic ability compared with conventional BMSCs, and can treat bone defects more effectively, shorten bone repair time and significantly improve the prognosis.

The stem cells and the preparation method therefor and use thereof, as well as a labeled composition and application thereof provided in the Examples herein have been described in detail above. Herein, specific examples are used to illustrate the principles and implementations of the disclosure. The description of the above Examples is only used to help understand the methods and core ideas of the present disclosure. At the same time, for a person skilled in the art, according to the ideas of the present disclosure, there may be changes in the specific implementations and application scopes. Therefore, the contents of this disclosure should not be understood as limiting the scope of the disclosure.

## Claims

1. A stem cell, wherein markers expressed by the stem cell comprise CX43 and FGFR2.

2. The stem cell according to claim 1, wherein the stem cell is CX43 positive and FGFR2 positive.

3. The stem cell according to claim 1 or 2, wherein the stem cell comprises a genetic marker selected from the group consisting of: FKBP11, PTN, TNN, TNC, OMD, ACTA2, MARCKS, and a combination thereof.

4. The stem cell according to any one of claims 1 to 3, wherein the stem cell is a mesenchymal stem cell.

5. The stem cell according to any one of claims 1 to 4, wherein the stem cell is derived from a human stem cell or an animal stem cell.

6. The stem cell according to claim 5, wherein the animal stem cell comprises any one of a deer-derived stem cell and a murine-derived stem cell.

7. The stem cell according to claim 6, wherein the deer-derived stem cell is derived from one or two of a mesenchymal tissue of antler blastemas or a mesenchymal tissue at a top of an antler growth center.

8. The stem cell according to claim 6 or 7, wherein the murine-derived stem cell is derived from a mouse P3 digit tip regeneration tissue.

9. The stem cell according to any one of claims 5 to 8, wherein the stem cell is induced by the human stem cell.

10. A method of preparing stem cells, comprising:
providing a cell population sample and an antibody composition, the antibody composition comprising an anti-CX43 antibody and an anti-FGFR2 antibody; and
staining the cell population sample with the antibody composition, and isolating cells positive for both CX43 and FGFR2 from the cell population sample to obtain stem cells.

11. The method according to claim 10, wherein the cell population sample is derived from animal stem cells or human stem cells.

12. The method according to claim 11, wherein the animal stem cells are derived from one or more of a mesenchymal tissue of antler blastemas, a mesenchymal tissue at a top of an antler growth center and a mouse P3 digit tip regeneration tissue.

13. The method according to any one of claims 10 to 12, wherein said "staining the cell population sample with the antibody composition, and isolating cells positive for both CX43 and FGFR2 from the cell population sample to obtain stem cells" comprises:
staining the cell population sample with one of the anti-CX43 antibody and the anti-FGFR2 antibody, and detecting and isolating positive cells in the cell population sample by flow cytometry to obtain primary screening cells; and
staining the primary screening cells with another one of the anti-CX43 antibody and the anti-FGFR2 antibody, and detecting and isolating positive cells in the primary screening cells by flow cytometry to obtain stem cells.

14. Use of stem cells in preparing a product for treating bone defect, wherein the stem cells comprise the stem cell according to any one of claims 1 to 9, or the stem cells comprise the stem cells prepared by the method according to any one of claims 10 to 13; and
wherein the product for treating bone defects comprises drugs for treating bone defects, materials for treating bone defects, or kits for treating bone defects.

15. The use according to claim 14, wherein the drugs for treating bone defects comprise the stem cells and a pharmaceutically acceptable carrier.

16. The use according to claim 14, wherein the materials for treating bone defects comprise any one of tissue engineering bone materials, bone defect scaffold materials, and bone defect composite repair materials.

17. The use according to claim 16, wherein the materials for treating bone defects comprise the stem cells and implantation materials, and the implantation materials comprise one or more of natural polymers, synthetic polymers, biomedical metal materials, or inorganic materials.

18. The use according to claim 16 or 17, wherein the bone defect scaffold materials comprise the stem cells and medical bone materials, and 1×10⁴ to 1×10⁵ of the stem cells are inoculated per cubic millimeter of the medical bone materials.

19. The use according to any one of claims 14 to 18, wherein an application subject of the product for treating bone defects is a human or an animal.

20. A method of treating bone defects, wherein the method comprises administering to a subject drugs, materials, or kits comprising an effective amount of stem cells, wherein the stem cells comprise the stem cell according to any one of claims 1 to 9, or the stem cells comprise stem cells prepared by the method according to any one of claims 10 to 13.
